# EUROPEAN PATENT APPLICATION

(11) **EP 3 599 465 A1**
(43) Date of publication of application: **29.01.2020**
(21) Application number: 18185239.3
(22) Date of filing: 24.07.2018
(51) Int. Cl.: G01N 33/50, A01K 43/04, C12Q 1/6879

(54) **METHOD FOR DETERMINING A SPECIFIC CHARACTERISTIC OF AN EMBRYO IN AN UNHATCHED EGG**

(71) Applicant: Tronico, 85660 Saint Philbert de Bouaine (FR); Centre National de la Recherche Scientifique - CNRS, 75016 Paris (FR)
(72) Inventor: Molina, Franck, 34270 Les Matelles (FR); Espeut, Julien, 34380 Saint Martin de Londres (FR)
(74) Representative: Touroude, Magali Linda

(57) **Abstract**

The present invention is directed to a method for determining whether a compound present at the surface of, or in the eggshell, is a biomarker specific of a characteristic of an embryo in an unhatched egg. The invention is also directed to a non-destructive method for the determination of a characteristic of an embryo, preferably the gender of an embryo, in an unhatched egg. The invention also relates to a kit to implement such a method.

SEQUENCE LISTING

<110> TRONICO

<120> METHOD FOR DETERMINING A SPECIFIC CHARACTERISTIC OF AN EMBRYO IN AN
UNHATCHED EGG

<130> TRO B001 EP1

<160> 43

<170> BiSSAP 1.3.6

<210> 1
<211> 21
<212> DNA
<213> Gallus gallus


<400> 1
gttactgatt cgtctacgag a
21


<210> 2
<211> 21
<212> DNA
<213> Gallus gallus


<400> 2
attgaaatga tccagtgctt g
21


<210> 3
<211> 20
<212> DNA
<213> Gallus gallus


<400> 3
gagatcacga actcaaccag
20


<210> 4
<211> 23
<212> DNA
<213> Gallus gallus


<400> 4
ccagacctaa tacggtttta cag
23


<210> 5
<211> 20
<212> DNA
<213> Gallus gallus

<400> 5
gactcttaga aacaggggca
20

<210> 6
<211> 20
<212> DNA
<213> Gallus gallus

<400> 6
gccatagatc tcacgaggag
20

<210> 7
<211> 22
<212> DNA
<213> Gallus gallus

<400> 7
cccaaatata acacgcttca ct
22

<210> 8
<211> 23
<212> DNA
<213> Gallus gallus

<400> 8
gaaatgaatt attttctggc gac
23

<210> 9
<211> 24
<212> DNA
<213> Gallus gallus

<400> 9
ctataatcga taatccacga ttca
24

<210> 10
<211> 23
<212> DNA
<213> Gallus gallus

<400> 10
cttgacctgt cttattagcg agg
23

<210> 11
<211> 20
<212> DNA
<213> Gallus gallus

<400> 11
agctctttct cgattccgtg
20

<210> 12
<211> 20
<212> DNA
<213> Gallus gallus

<400> 12
gggtagacac aagctgagcc
20

<210> 13
<211> 447
<212> DNA
<213> Gallus gallus

<400> 13
gttactgatt cgtctacgag aacgtggcaa cagagtactg attttctctc agatggtgag 60

gatgctagac atcctagcag aatacttgaa gtatcgtcag tttccttttc aggtaagaat 120

tttgatggta gtagccaaga agccttgatc tttgccactt tatcttaagt aaaagtgtcc 180

tttctgtaga aaagacttct aaaagtttaa ttttatgtat agaaaaagac tggcaattac 240

tatggtgtga ggtgttgcat tattctcctc ctcctccttc ccccccattc ctccccttgc 300

cctcagttgt tttggcaatt gagtattcag gttgctctga ttagaatata gtatgagttc 360

ctttttaact gtaatatttg atctctttag agacttgatg gatcaataaa aggagaactg 420

aggaaacaag cactggatca tttcaat 447

<210> 14
<211> 211
<212> DNA
<213> Gallus gallus

<400> 14
gagatcacga actcaaccag ggcatggtca ctgcagccca ggctgcctcc aaccaacacc 60

tttaatgatc tcctccgcat tggtgagcag caggtccagc aacgcttcac ctctggtcgg 120

tctgtccaat acctgaacca gaaagttatc atcaatggat tccagggcct cctggatctc 180

ttgcagcctg taaaaccgta ttaggtctgg a 211

<210> 15
<211> 326
<212> DNA
<213> Gallus gallus

<400> 15
gactcttaga aacaggggca acagtcatcc cacaactaat tggcaaatgg ctctcagaaa 60

ttatagtgca aactccagtt tttgtagcta aggggacatt aatcatatca ttatggcaaa 120

ctggaccccc ccctttggta cctgatatag taatgcaacc atcaacatca ggccaaaggg 180

tatggtataa gagacctggc atgctgctgt gcaagcagaa atattaacgc aagatagaaa 240

taccatgtgc attctaccgc ggatagtggt cttgcccata ctggtaccaa ctaaacactt 300

attctactcc tcgtgagatc tatggc 326


<210> 16
<211> 416
<212> DNA
<213> Gallus gallus


<400> 16
cccaaatata acacgcttca ctcacaaagc acgcattttc accctgaagt accacctttc 60

agccgaaaat tacgcttttt cctccagaaa ataccacttc tcaaacagaa atatcacgtt 120

ttcgccaaga aaatagcacc attcacccca aaatcacgcg ttttctctcc agaactacca 180

cttttctcac agaaatcaca cattttcttc ccgaaagtac caccttgcac acgaaaatca 240

cgcattttct gcgcgaaaca accccatttc accccaaaat cagtcttttt cttccgaaaa 300

atacaacttt tctaacggaa accatgcact gtcactccga aaatacaagt ttttgcccga 360

aaatcacgca ttttcccttc gaaaataccc cacgtcgcca gaaaataatt catttc 416


<210> 17
<211> 131
<212> DNA
<213> Gallus gallus


<400> 17
ctataatcga taatccacga ttcacccaac caccccttgc cagcacagcc tacataccgc 60

cgtcgccagc ccacctctaa tgaaagaaca acagtgagct caatagcccc tcgctaataa 120

gacaggtcaa g 131


<210> 18
<211> 256
<212> DNA
<213> Gallus gallus


<400> 18
agctctttct cgattccgtg ggtggtggtg catggccgtt cttagttggt ggagcgattt 60

gtctggttaa ttccgataac gaacgagact ctggcatgct aactagttac gcgacccccg 120

agcggtcggc gtccaacttc ttagagggac aagtggcgtt cagccacccg agattgagca 180

ataacaggtc tgtgatgccc ttagatgtcc ggggctgcac gcgcgctaca ctgactggct 240

cagcttgtgt ctaccc 256

<210> 19
<211> 12
<212> PRT
<213> Gallus gallus

<400> 19
Asp Asp Asp Lys Asn Leu Gly Ala Leu Cys Gly Arg
1               5                   10

<210> 20
<211> 20
<212> PRT
<213> Gallus gallus

<400> 20
Cys Asn Ser His Gly Tyr Ile Glu Glu Ala Cys Ile Ile Pro Cys Pro
1               5                   10                  15

Ser Asp Cys Lys
            20

<210> 21
<211> 17
<212> PRT
<213> Gallus gallus

<400> 21
Val Tyr Pro Glu Thr Gln Ala Gln Glu Pro Gly Val Ser Ala Ser Leu
1               5                   10                  15
Lys

<210> 22
<211> 14
<212> PRT
<213> Gallus gallus

<400> 22
Val Pro Val Thr Asp His Ser Leu His Leu Ser Gln Ala Lys
1               5                   10

<210> 23
<211> 15
<212> PRT
<213> Gallus gallus

<400> 23

Ser Tyr Lys Pro Pro Phe Asp Thr Phe Glu Tyr Phe Gln Asp Lys
1               5                   10                  15

<210> 24
<211> 15
<212> PRT
<213> Gallus gallus


<400> 24
Thr Leu Phe Gly Gln Tyr Gly Gly Gly Ser Phe Ser Phe Ser Arg
1               5                   10                  15

<210> 25
<211> 12
<212> PRT
<213> Gallus gallus


<400> 25
Arg Gly Gly Gly Pro Gln Ala Ile Ser Ile Gly Lys
1               5                   10

<210> 26
<211> 28
<212> PRT
<213> Gallus gallus


<400> 26
Asp Gly Asn Ala Ser Gly Thr Thr Leu Leu Glu Ala Leu Asp Cys Ile
1               5                   10                  15
Leu Pro Pro Thr Arg Pro Thr Asp Lys Pro Leu Arg
            20                  25

<210> 27
<211> 19
<212> PRT
<213> Gallus gallus


<400> 27
Thr Gln Val Glu Thr Leu Glu Glu Asp Tyr Thr Phe Met Ala Leu Ser
1               5                   10                  15
Leu Trp Lys


<210> 28
<211> 8
<212> PRT
<213> Gallus gallus


<400> 28
Ile Thr Val Val Ala Ala Asp Arg
1               5

<210> 29
<211> 10
<212> PRT
<213> Gallus gallus

<400> 29
Leu Val Gln Ala Phe Gln Phe Thr Asp Lys
1               5                   10

<210> 30
<211> 29
<212> PRT
<213> Gallus gallus

<400> 30
Glu Asp Glu Asn Ile Leu Phe Cys Pro Leu Ser Ile Ala Ile Ala Met
1               5                   10                  15
Gly Met Ile Glu Leu Gly Ala His Gly Thr Thr Leu Lys
            20                  25

<210> 31
<211> 13
<212> PRT
<213> Gallus gallus

<400> 31
Cys Pro Gly Glu Ser Ser Gln Ile Cys Asp Trp Ile Arg
1               5                   10

<210> 32
<211> 13
<212> PRT
<213> Gallus gallus

<400> 32
Glu Leu Lys Pro Thr Val Val Asn Leu Asp Phe Gln Arg
1               5                   10

<210> 33
<211> 12
<212> PRT
<213> Gallus gallus

<400> 33
Leu His Leu Asp Leu Ala Val Asp Val Val Ala Lys
1               5                   10

<210> 34
<211> 15
<212> PRT
<213> Gallus gallus

<400> 34
Ile Ala Gly His Ala Glu Glu Tyr Gly Ala Glu Thr Leu Glu Arg
1               5                   10                  15

<210> 35
<211> 16
<212> PRT
<213> Gallus gallus

<400> 35

Gly Ala Thr Tyr Asn Ile Ile Val Glu Ala Leu Lys Asp His Arg Arg
1               5                   10                  15

<210> 36
<211> 10
<212> PRT
<213> Gallus gallus

<400> 36
Val Leu Thr Ser Phe Gly Asp Ala Val Lys
1               5                   10

<210> 37
<211> 27
<212> PRT
<213> Gallus gallus

<400> 37
Leu Ser Pro Ser Phe Gln Gly His Val Cys Gly Leu Cys Gly Asn Tyr
1               5                   10                  15
Asp Gly Asn Gly Asn Asn Asp Phe Thr Thr Arg
            20                  25

<210> 38
<211> 10
<212> PRT
<213> Gallus gallus

<400> 38
Ser Arg Pro Ile Leu Pro Ile Tyr Leu Lys
1               5                   10

<210> 39
<211> 17
<212> PRT
<213> Gallus gallus

<400> 39
Ala Leu His Phe Asp Ser Ile Ala Gly Leu Gly Gly Ser Thr Gln Thr
1               5                   10                  15
Lys

<210> 40
<211> 10
<212> PRT
<213> Gallus gallus

<400> 40
Glu Leu Leu Ser Asp Ile Thr Ala Ser Lys
1               5                   10

<210> 41
<211> 12
<212> PRT
<213> Gallus gallus

<400> 41
His Asn Pro Thr Asn Thr Ile Val Tyr Phe Gly Arg
1               5                   10

<210> 42
<211> 8
<212> PRT
<213> Gallus gallus


<400> 42
Phe Gly Val Asn Gly Ser Glu Lys
1               5

<210> 43
<211> 9
<212> PRT
<213> Gallus gallus


<400> 43
Pro Gln Ala Pro Ile Thr Gly Tyr Arg
1               5

## Description

The present invention relates to a method for determining whether a compound or a combination of compounds present at the surface of, or in the eggshell is a biomarker specific of a characteristic of an embryo in an unhatched egg. The invention is also directed to a method for the determination of gender, fertility, stages of development, viability or any genetic or metabolic characteristics of interest of an embryo in an unhatched egg using said biomarker.

For commercial egg production of avian species, the incubation and rearing of male chicks is highly undesirable, leading to the elimination of male chicks. In addition, in hatchery management, it may be desirable to separate the populations to gain feed efficiencies, improve processing uniformity, and to reduce production costs where there are differences in growth rate and nutritional requirements of male and female birds.

Moreover, there is a percentage of eggs that are unfertilized, or do not comprise a viable embryo at the beginning of the incubation period, which greatly reduces the capacity of the incubators at hatcheries.

Accordingly, it would be of great value to provide a method which is non-destructive and allows to determine in an unhatched egg and in an early stage the gender and/or other characteristics as for instance fertility, stages of development, viability or any genetic or metabolic characteristics of interest, of avian embryos.

Several approaches have been used to determine the gender of fertile eggs based on molecular and hormone assays that are laborious and invasive in nature. The techniques are of limited use in the industry as they are unsuitable for automated, high throughput applications.

For example, the following documents can be cited:
- The US Patent Application published under the number 201502607,0 filed on September 17, 2015 (Bruins W.S. et al.) discloses a process for the non-destructive determination of gender of an avian embryo in an egg, comprising the detecting at least a first developmental marker compound selected from sugars and/or amino acids, precursors and metabolites thereof from a sample of the allantoic fluid of the egg.
- The US Patent 8,624,190 dated January 7, 2014 (Steiner et al.) discloses a method and device for determining the sex of fertilized, non-incubated bird eggs where a probe for measuring a spectrum is introduced through a hole of the egg shell towards the blastodisc and where spectroscopic in-ovo characterization of the blastodisc cells are automatically classified and the sex identified according to the classification obtained.
- The US Patent Application published under the No. 20180033139 and dated February 1, 2018 (Ngadi M. et al) discloses systems, devices, and methods for detecting characteristics of an unhatched egg wherein a set of images of an unhatched egg are obtained, where each of the spectral images is obtained in a particular wavelength range, and wherein the set of images is processed to extract image features, and to classify the unhatched egg according to at least one characteristic including fertility and/or gender.
- the document PLoS One. 2018; 13(2): e0192554 (Galli R.), Published online on 23 February 2018 which discloses a method of sexing of chicken eggs by fluorescence and Raman spectroscopy through the shell membrane which can be performed on perfused extraembryonic vessels localized immediately under the membrane.

Therefore, there is a need for improved technology for detecting a specific embryo characteristic such as gender or fertility in unhatched eggs.
This is the subject of the present invention.

Surprisingly and contrary to what has sometimes be argued, it has been demonstrated by the inventors that shell egg contains specific markers of the embryo.
Furthermore, the inventors have also demonstrated that some of these markers, alone or in combination, can be used as specific biomarker of the embryo including for example, but not limited to, gender, fertility, stages of development, viability or any genetic or metabolic characteristics of interest.

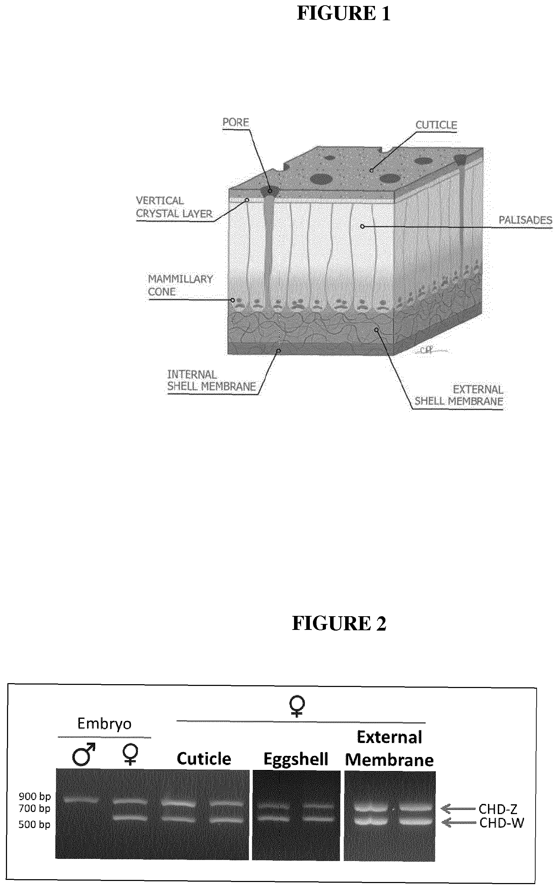

Accordingly, the present invention relates to a method for determining whether a compound present at the surface of, or in the eggshell is a biomarker specific of an embryo, preferably of an alive embryo, in an unhatched egg, said method comprising the steps of:
a) determining the presence of at least one compound in or on the eggshell or, said measure being carried out at different time periods from the incubation until to the hatching of the egg;
b) optionally measuring the amount of said compound in or on the eggshell, said measure being carried out at different time periods of from the incubation until to the hatching of the egg;
c) comparing the results obtained in step a) and, optionally in step b), particularly the decrease or the increase of the compound amount obtained in step b) function of the location of the analysed egg sample, to a base line established for said compound for positive and negative controlled embryos for said characteristic;
d) determining whether said compound is a biomarker specific of a characteristic of the embryo.
According to the invention, the method further includes a step e) of selecting the tested compound as a specific embryo biomarker for said characteristic.
As represented in Figure 1 (Hincke et al Frontiers in Biosciences, 2012), typical poultry eggshell is made of a combination of intricated sub-structures: cuticle, pores, vertical crystal layer, palisades, mammillary cones, external shell membrane and internal shell membrane.
In this way, by extension, in the present description, the term "eggshell" as used herein, includes said intricated sub-structures.

In a further aspect, the present invention relates to a method for the determination of a characteristic of an embryo in an unhatched egg, said method comprising the steps of:
a) detecting and/or measuring the amount of at least a known biomarker specific of said characteristic at a time period of from the incubation from the egg laying until to the hatching of the egg;
b) comparing the amount obtained in step a) to a base line established for said biomarker at the same time incubation period for positive and negative controlled embryos for said characteristic;
c) determining whether the embryo possesses said characteristic by analysing the results obtained in step b),
   and wherein the amount of said biomarker in detecting at the surface or in the eggshell of the egg.

Preferably the detection or measurement is performed in step a) at a period of from 1 to 15 days, more preferably of from 2 to 14, yet more preferably of from 3 to 13, and even more preferably of from 4 to 12 days after the incubation is started, such as performing step a) preferably at a time period of from 4 to 7 days after the beginning of the incubation of the egg.

Preferably, in the method of the present invention, at least a second compound or biomarker is detected and/or measuring in step (a), and wherein the at least first and second compounds or biomarker are analysed and compared to the base line established for said two compounds or biomarkers.

By correlating the analysis of at least two biomarkers, the embryo characteristic which is desired to determine may advantageously be more selective and thus the method be improved.

Preferably the method for the determination of a characteristic of an embryo in an unhatched egg, according to the present invention is performed as a non-destructive method.

By the term " non-destructive method", it is intended to designate a method allowing the tested avian embryo to grow or to remain viable during the incubation step. Preferably the method for the determination of a characteristic of an embryo in an unhatched egg, according to the present invention is performed by a method wherein the eggshell inner membrane has not been penetrated.

According to a preferred embodiment, said known biomarker which is detected and/or measuring is a biomarker selected by the method of the present invention.

According to further features in preferred embodiments of the invention described, said compound or biomarker which is detected and/or measured is selected from the group consisting of nucleic acids, preferably ADN, proteins, peptides and metabolites. In a preferred embodiment, in the method of the present invention, said egg is selected from the group of oviparous animals, including birds.

In a more preferred embodiment, said egg is selected from the group of avian egg, preferably poultry eggs, more preferably selected from the group consisting of chicken, ducks, turkeys, geese, pheasant and quail.

According to further features in preferred embodiments of the invention described, said embryo characteristic which is desired to determine is selected from the group consisting of gender, fertility, stages of development, viability or any genetic or metabolic characteristics of interest.

More preferably, said embryo characteristic which is desired to determine is the gender of the embryo.

According to further features in preferred embodiments of the invention described, the measure of said biomarker compound is carried out on eggshell or targeting at least one of the egg shell sub-structure selected from the group consisting of the cuticle, the pores, the vertical crystal layer, the palisades, the mammillary cones, the external shell membrane and the inner shell membrane (see figure 1).

According to still further features in preferred embodiments of the invention described, said compound is a nucleic acid biomarker compound, preferably DNA fragment, more preferably a nucleic acid whose presence or concentration is specific of the embryo gender.

In a more preferred embodiment, said specific DNA fragment allowing the embryo gender determination of the egg is selected from the group of W-linked genic or intergenic regions HINTW, CHD1-W, AC239603.4, AC182258.2, AF461428.1, SWIM, ATP5A1W, Spin-W, ASW, Sox9, CYP19A1, AMH (Anti-Mullerian hormone), FET1(female expressed transcript; trans-membrane domain), SF1 and Dax. In a more preferred embodiment, said specific DNA fragment allowing the gender determination of the egg is the CHD1-W, SWIM gene or AC239603.4, AC182258.2, AF461428.1 or specific fragments thereof fragments.

Most preferred are DNA fragment having or comprising a sequence selected from the group consisting of SEQ ID NO. 13 to 18, or comprising a nucleic fragment of the sequence SEQ ID NO. 13 to 18, said specific fragment having at least 12, preferably 13, 14, 15 or 16 nucleotides.

According to still further features in preferred embodiments of the invention described, said compound is a protein or peptide biomarker whose presence or concentration is specific of the embryo gender.

Most preferred are proteins or peptides biomarker having or comprising an amino acid sequence selected from the group consisting of the sequences SEQ ID NO. 19 to 43, preferably a protein, or a peptide comprising at least 8, preferably 9, 10, 11, 12, 13, 14 or 15 consecutive amino acids of the sequences SEQ ID NO. 19 to 43.

In another preferred embodiment, said compound is a metabolite biomarker compound. Preferably the glucose, whose presence or concentration is specific of the embryo gender.

According to further features in preferred embodiments of the invention described, the measure of said biomarker compound is measured by a method selected from the group of methods consisting of : PCR or isothermal PCR, Q-PCR or colorimetric, fluorescent, luminescent, electrochemical or electromagnetic signal, magnetic resonance spectroscopy, Infra-red spectroscopy, Raman spectroscopy, liquid or gaz-chromatography, mass spectrometry, depending on the nature of the biomarker that has to be detected and/or measured (nucleic acid, peptides or proteins or metabolites)

Preferably, the biomarker compound may be detected or measured by any method suitable to detect and to quantify said specific biomarker and performed non-destructively and more preferably by method wherein the total or part of the eggshell, particularly the inner membrane therein, remain imperforated.

In a preferred embodiment, the biomarker compound is measured by a combination of biotechnological methods including synthetic biology or artificial bio-molecular networks designed to detect and measure biomarkers on eggshell or eggshell sub-structure.

More particularly, the invention concerns a kit for the determination of the presence, the level, the location of the biomarker which is desired to detect and measure on eggshell or eggshell sub-structure.

In a preferred embodiment, as it will be described in the examples, the invention relates to a kit for detecting and measuring on eggshell or eggshell sub-structure specific nucleic or protein or peptides biomarkers.

In another aspect, the present invention is directed to a kit for the determination of a characteristic of an egg embryo, preferably of the egg embryo gender, wherein said kit comprises at least:
- a DNA fragment, or complementary and/or labelled fragment thereof, able to specifically hybridize or recognize the nucleic biomarker which is desired to detect and measure on eggshell or eggshell sub-structure.

In a preferred embodiment, said kit for the determination of a characteristic of an egg embryo, preferably of the egg embryo gender, comprises at least:
- a DNA fragment, or complementary and/or labelled fragment thereof, able to specifically hybridize or recognize the nucleic biomarker which is desired to detect and measure on eggshell or eggshell sub-structure, and selected from the group consisting of DNA fragment, or complementary sequence thereof, having or comprising a sequence selected from the group of the sequences the SEQ ID NO. 13 to 18, or comprising a nucleic fragment of the sequence SEQ ID NO. 13 to 18 having at least 12, preferably 13, 14, 15 or 16 nucleotides.

In a more preferred embodiment, said kit for the determination of a characteristic of an egg embryo, preferably of the egg embryo gender, comprises at least one pair of primers selected from the group of :
SEQ ID NO.1 and 2; SEQ ID NO.3 and 4; SEQ ID NO. 5 and 6; SEQ ID NO. 7 and 8, SEQ ID NO. 9 and 10; and SEQ ID NO. 11 and 12.

In a particular embodiment, the invention consists in a kit comprising at least an antibody directed specifically to the protein or peptide biomarker which is desired to detect and measure on eggshell or eggshell sub-structure.

Depending of the number of protein or peptide biomarker which are desired to detect and measure on eggshell or eggshell sub-structure, for example 1, 2, 3 or 4, said kit can comprise 1, 2, 3 or 4 specific antibodies directing against (capable of recognizing) the proteic or peptidic desired biomarker.

Monoclonal or polyclonal antibodies or binding fragments thereof can be provided labelled with a detectable moiety, such that they may be packaged and used, for example, in kits, to identify and to measure on eggshell or eggshell sub-structure the eggs having the aforementioned protein or peptide biomarker.

In general, for the preparation of monoclonal antibodies or their functional fragments or derivatives, especially of murine origin, it is possible to refer to techniques which are described in particular in the manual "Antibodies" (Harlow and Lane, Antibodies: A Laboratory Manual, Cold Spring Harbor Laboratory, Cold Spring Harbor NY, pp. 726, 1988) or to the technique of preparation from hybridomas described by Kohler and Milstein (Nature, 256:495-497, 1975).

The monoclonal antibodies can be obtained, for example, from an animal cell immunized against the proteic or peptidic biomarker desired to be identified and measured, or one of its fragments containing the specific epitope.

In another preferred embodiment, the kit of the invention comprises a reagent useful for quantifying the level of binding between the labelled antibody and the specific protein or peptide biomarker.

For example, the kit according to the invention useful for determining specific protein or peptide biomarker comprises:
i) a reagent useful for detecting the extent of binding between the said labelled antibody and said protein or peptide specific biomarker ; and, optionally,
ii) positive and negative control samples.

In a more preferred embodiment, the kit of the invention comprises a reagent useful for quantifying the level of binding between the labelled antibody and the specific protein or peptide biomarker and wherein said protein or peptide biomarker is selected from the group consisting of the sequences SEQ ID NO. 19 to 43, preferably a protein, or a peptide comprising at least 8, preferably 9, 10, 11, 12, 13, 14 or 15 consecutive amino acids of the sequences SEQ ID NO. 19 to 43.

In a more preferred embodiment, the kit of the invention comprises a reagent useful for quantifying the level of binding between at least two, preferably 3 or 4 antibodies, optionally labelled, and two, preferably 3 or 4, specific protein or peptide biomarkers and wherein said at least two, preferably 3 or 4 proteins or peptide biomarkers are selected from the group consisting of the sequences SEQ ID NO. 19 to 43, preferably proteins, or peptides comprising at least 8, preferably 9, 10, 11, 12, 13, 14 or 15 consecutive amino acids of the sequences SEQ ID NO. 19 to 43.

For example, the kit according to the invention useful for determining specific protein or peptide biomarker comprises :
i) a reagent useful for detecting the extent of binding between the said labelled antibody and said protein or peptide specific biomarker ; and, optionally,
ii) positive and negative control samples.

Non-limiting examples of such labels include fluorophores such as fluorescein isothiocyanate; chromophores, radionuclides, or enzymes. Such labelled antibodies or binding fragments may be used for the determination of the presence, the level or the localization of the desired protein or peptide biomarker on eggshell or eggshell sub-structure. ELISA, as well as other immunological techniques for detecting or quantifying eggshell or eggshell sub-structure exhibiting said desired biomarker.

Thus, kits can be provided which contain the antibodies for detection and quantitation the desired protein or peptide biomarker in an ELISA or a Western blot. As with the article of manufacture, the kit comprises a container and a label or package insert on or associated with the container. The container holds a composition comprising at least one antibody or binding fragment thereof. Additional containers may be included that contain, e.g., diluents and buffers, and control. The label or package insert may provide a description of the composition as well as instructions for the intended diagnostic use. It must be understood that any labelling method can be used by the man skilled in the art such as, for example, the use of labels above mentioned.

Thus, the antibody, or a functional fragment thereof, can be employed in a process for the detection and/or the quantification of the desired protein or peptide biomarker on eggshell or eggshell sub-structure, characterized in that it comprises the following steps:
a) the contacting of the eggshell or eggshell sub-structure exhibiting said desired biomarker with an antibody, or a functional fragment thereof, without penetrated the inner membrane of the eggshell; and
b) the demonstration of the desired protein or peptide biomarker /antibody complex possibly formed.

Any procedure or conventional test can be employed in order to carry out such a detection and/or dosage. Said test can be a competition or sandwich test, or any test known to the person skilled in the art dependent on the formation of an immune complex of antibody-antigen type. Following the applications according to the invention, the antibody or a functional fragment thereof can be labelled.

By way of example, a preferred method brings into play immunoenzymatic processes according to the ELISA technique, by immunofluorescence, or radioimmunoassay (RIA) technique or equivalent.

Thus, the present invention likewise comprises the kits or sets necessary for carrying out the methods of the present invention, preferably for the determination of a characteristic of an embryo in an unhatched egg, preferably the gender of the embryo, more preferably without penetrating the inner membrane of the eggshell, characterized in that said kit comprises the following elements:
a) a pair of primers or an antibody specific/able to hybridizing/recognizing of the desired DNA sequence, proteic or peptidic, or metabolic biomarker on eggshell or eggshell sub-structure, which is desired to identify or to measure, preferably wherein :
   - the pair of primers is selected from the group of pair of primers having the sequences SEQ ID NO. 1 and 2; SEQ ID NO. 3 and 4; SEQ ID NO. 5 and 6; SEQ ID NO. 7 and 8; SEQ ID NO. 9 and 10; and SEQ ID NO. 11 and 12,
      or
   - said antibody is an antibody able to specifically recognizing the protein or peptide biomarker selected from the group consisting of the sequences SEQ ID NO. 19 to 43, preferably a protein, or a peptide comprising at least 8, preferably 9, 10, 11, 12, 13, 14 or 15 consecutive amino acids of the sequences SEQ ID NO. 19 to 43;
b) optionally, the reagents for the formation of the medium favorable to the formation of the complex between the primer or antibody and the desired biomarker ; and
c) optionally, the reagents allowing the demonstration of the presence and/or the quantification of the complexes.

The following examples, the figures and the legends hereinafter have been chosen to provide those skilled in the art with a complete description in order to be able to implement and use the present invention These examples are not intended to limit the scope of what the inventor considers to be its invention, nor are they intended to show that only the experiments hereinafter were carried out.

Other characteristics and advantages of the invention will emerge in the remainder of the description with the Examples and Figures, for which the legends are given hereinbelow.

### Figure legends:

**Figure 1:** Figure 1 depicts typical chicken eggshell made of a combination of intricated sub-structures cuticle, pores, vertical crystal layer, palisades, mammillary cones, external shell membrane and internal shell membrane.
**Figure 2:** Figure 2 depicts an example CHD-W and CHD-Z fragments that have been amplified and detected at different places in the external layer of the egg (Cuticle, Eggshell and External membrane)

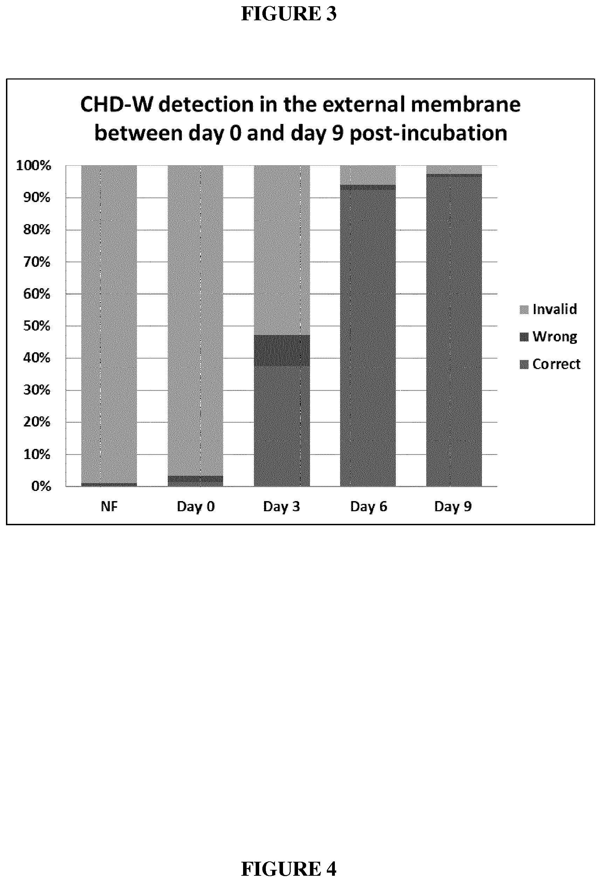

**Figure 3****:** CHD-W female specific biomarker is appearing in the external membrane with time after incubation. CHD-W is absent in non-fertilized eggs (NF), absent at day 0, present in 38% of the eggs at day 3 of incubation, present in 93% of the eggs at day 6 of incubation and present in 97% of the eggs at day 9 of incubation. Invalid: No DNA fragment amplified, Wrong: CHD-W signal amplified for male egg, Correct: CHD-W fragment amplified for female eggs and NO CHD-W fragment amplified for male eggs.

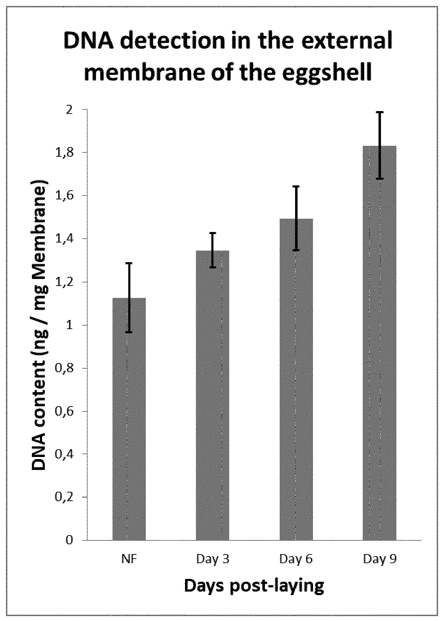

**Figure 4****:** Total DNA content in the external membrane of the eggshell is increasing with time. The DNA content in the external membrane is at around 1.12 ng per mg of tissue in non-fertilized eggs and it increases with time for fertilized eggs. DNA specific of the embryo developing inside is accumulating with time in the external membrane.NF: Non-fertilized eggs; Day 3, 6, 9: Days post-laying for fertilized eggs.

### EXAMPLE 1: Material and Methods

### A) Eggs Incubation

Fertilized or unfertilized ISA Brown eggs were obtained from a commercial supplier (SFPA, Saint-Marcellin France). Eggs were stocked for a maximum of 10 days after laying at 19°C. Fertile eggs were incubated in a dedicated incubator (Masson, Soyans France) at 37.5 °C, 55% humidity and tilted every hour for 9 days.

### B) Samples Collection and Lysis for Eggshell DNA Detection

After egg opening and removing of the membranes bound to the eggshell, eggshells were mechanically dissociated using pestle and mortar. For each extraction, 80 mg of eggshell powder was incubated overnight at 56°C under vigorous shaking in 900 ml of lysis buffer (20 mM Tris-HCl pH 8, 0.47 M EDTA-2Na, 1% Triton X-100, 10 mM DTT, 50 µg proteinase K). Eggshell lysates were centrifugated for 10 minutes at 2000g at RT°. Supernatants buffer was exchanged with 20 mM Tris buffer at pH8 with NAP-10 column (Biorad) and samples were concentrated by precipitating the DNA with 100% ethanol.

### C) Samples Collection and Lysis for Membrane DNA Detection

After egg opening, inner and external membranes were dissociated from the eggshell. We used the QIAmp DNA Micro Kit for DNA extraction with some variation compared to the original protocol. For each sample, 150 mg of membrane was lysed in 500 µl of ATL Buffer, containing 40 µg of proteinase K and 5 mM of DTT, overnight at 56°C under vigorous shaking. After centrifugation at 3000 g for 3 minutes, 400 µl of buffer AL and 400 µl of 100% ethanol were added to the supernatant. The entire lysate was transferred to QIAamp MinElute column and centrifugated at 6000 g for 1 minute. After washing with AW1 and AW2 buffers, the DNA was eluted with 60 µl of AE buffer.

### D) Membranes and Eggshell DNA Detection by PCR

Membrane and eggshell DNA samples were amplified by PCR in order to detect specific DNA sequences. For the PCR we used the "Invitrogen Platinum Green Hot Start PCR kit". 1 µl of lysate was mixed with 9.5 µL nuclease free water, 12.5 µL of 2X Master Mix, 200 nM of primer. Amplification was performed by using a peqSTAR 96X thermocycler (Ozyme, Montigny-le-Bretonneux France). Thermal cycling conditions for DNA amplification were: 1 cycle of initial denaturation at 94°C for 2 minutes; 35 cycles comprising 30s at 94°C for the denaturation, 30s at 55°C for annealing, 30s at 72°C for the elongation; and a final extension cycle at 72°C for 5 minutes.

PCR products were analysed by regular agarose gel electrophoresis and microfluidic capillary electrophoresis systems (Caliper LifeSciences, France).

### E) List of PCR Primers Used for DNA Sequence Amplifications.

**Table 1: List of PCR Primers**

| **Primer name** | **Sequence targeted** | **Sequence** | **SEQ ID NO.:** | **Amplicon size** |
|---|---|---|---|---|
| CHD-F | CHD | GTTACTGATTCGTCTACGAGA | 1 | 447 bp |
| CHD-R | | ATTGAAATGATCCAGTGCTTG | 2 | |
| AC23-F | AC239603.4 | GAGATCACGAACTCAACCAG | 3 | 211 bp |
| AC23-R | | CCAGACCTAATACGGTTTTACAG | 4 | |
| AC18-F | AC182258.2 | GACTCTTAGAAACAGGGGCA | 5 | 326 bp |
| AC18-R | | GCCATAGATCTCACGAGGAG | 6 | |
| AF46-F | AF461428.1 | CCCAAATATAACACGCTTCACT | 7 | 416 bp |
| AF46-R | | GAAATGAATTATTTTCTGGCGAC | 8 | |
| 12S-F | 12S | CTATAATCGATAATCCACGATTCA | 9 | 131 bp |
| 12S-R | | CTTGACCTGTCTTATTAGCGAGG | 10 | |
| 18S-F | 18S | AGCTCTTTCTCGATTCCGTG | 11 | 256 bp |
| 18S-R | | GGGTAGACACAAGCTGAGCC | 12 | |

### F) DNA Sequences Amplified by the PCR Primers.

**CHD W (amplify 2 specific sequences: a male and female sequence on the Z-chromosome, plus a female sequence on the W-chromosome).** 
gttactgattcgtctacgagaacgtggcaacagagtactgattttctctcagatggtgaggatgctagacatcctagcagaat
acttgaagtatcgtcagtttccttttcaggtaagaattttgatggtagtagccaagaagccttgatctttgccactttatcttaagt
aaaagtgtcctttctgtagaaaagacttctaaaagtttaattttatgtatagaaaaagactggcaattactatggtgaggtgtt
gcattattctcctcctcctccttccccccattcctccccttgccctcagttgttttggcaattgagtattcaggttgctctgatta
gaatatagtatgagttcctttttaactgtaatatttgatctctttagagacttgatggatcaataaaaggagaactgaggaaaca
agcactggatcatttcaat (SEQ ID NO.: 13)
**AC239603.4 (amplify a female-specific sequence on the W-chromosome).** 
gagatcacgaactcaaccagggcatggtcactgcagcccaggctgcctccaaccaacacctttaatgatctcctccgcatt
ggtgagcagcaggtccagcaacgcttcacctctggtcggtctgtccaatacctgaaccagaaagttatcatcaatggattc
cagggcctcctggatctcttgcagcctgtaaaaccgtattaggtctgga (SEQ ID NO.: 14)
**AC182258.2 (amplify a female-specific sequence on the W-chromosome).** 
gactcttagaaacaggggcaacagtcatcccacaactaattggcaaatggctctcagaaattatagtgcaaactccagttttt
gtagctaaggggacattaatcatatcattatggcaaactggaccccccctttggtacctgatatagtaatgcaaccatcaac
atcaggccaaagggtatggtataagagacctggcatgctgctgtgcaagcagaaatattaacgcaagatagaaataccat
gtgcattctaccgcggatagtggtcttgcccatactggtaccaactaaacacttattctactcctcgtgagatctatggc
(SEQ ID NO.: 15)
**AF461428.1 (amplify a female-specific sequence on the W-chromosome).** 
ccaaatataacacgcttcactcacaaagcacgcattttcaccctgaagtaccacctttcagccgaaaattacgctttttcctcc
agaaaataccacttctcaaacagaaatatcacgttttcgccaagaaaatagcaccattcaccccaaaatcacgcgttttctct
ccagaactaccacttttctcacagaaatcacacattttcttcccgaaagtaccaccttgcacacgaaaatcacgcattttctgc
gcgaaacaaccccatttcaccccaaaatcagtctttttcttccgaaaaatacaacttttctaacggaaaccatgcactgtcact
ccgaaaatacaagtttttgcccgaaaatcacgcattttcccttcgaaaataccccacgtcgccagaaaataattcatttc
(SEQ ID NO.: 16)
**12S - MF041985.1 (amplify a common sequence for males and females which is part of 12S ribosomal RNA gene, mitochondrial DNA)** 
ctataatcgataatccacgattcacccaaccaccccttgccagcacagcctacataccgccgtcgccagcccacctctaat
gaaagaacaacagtgagctcaatagcccctcgctaataagacaggtcaag (SEQ ID NO.: 17)
**18S - (amplify a common sequence for males and females which is part of 18S ribosomal RNA gene, mitochondrial DNA)**

Agctctttctcgattccgtgggtggtggtgcatggccgttcttagttggtggagcgatttgtctggttaattccgataacgaac
gagactctggcatgctaactagttacgcgacccccgagcggtcggcgtccaacttcttagagggacaagtggcgttcagc
cacccgagattgagcaataacaggtctgtgatgcccttagatgtccggggctgcacgcgcgctacactgactggctcagc
ttgtgtctaccc (SEQ ID NO.: 18)

### G) Samples Collection and Lysis for Eggshell Protein and Peptides Detection

After egg opening and removing of the membranes bound to the eggshell, eggshells were mechanically dissociated using pestle and mortar. For each extraction, 250 mg of eggshell powder was incubated overnight at 4°C under shaking in 5 ml of demineralizing buffer (20% acetic acid). After centrifugation at 2000g for 2 minutes, the resulting suspensions was dialysed (cut off 3500 Da) against 0.4 M NaCl, 25 mM Na-Acetate, 0.1% Tween 20 at pH 7.4 for 24h at 4°C. For protein extraction and concentration, we performed a TCA (trichloro-acetic) precipitation and resuspended protein pellet in 100 µl of 8 M urea, 10 mM DTT, 50 mM Ammonium Bicarbonate. Samples were then reduced, alkylated, trypsin-digested and zip-tipped. Mass-spectrometry protein identification was performed on a Orbitrap Q Exactive HF spectrometer and primary peptides analysis was done with MaxQuant. Differential statistical analysis was done between males and females outputs at days 0 and 9 post-incubation with in house developed programs. Basically for one protein or peptide identified, the AUC (Area Under the Curve) of the ROC (Receiver Operating Characteristic) curve and fold change between females and males proteins or peptides were calculated. We deduced the sensitivity and specificity of our tests with Younden approaches using the R package pROC.

### H) Metabolites Detection in Eggshell

After incubation, the eggs were directly immerged in a solvent mix (H2O / Acetonitrile / Methanol 40/30/30) for 1h on air-chamber side. The metabolites extracted were submitted to liquid chromatography on a waters acquity HSS T3 column (150 mm X 2.1 mm and 1.8 µm) and detected with a Q-Exactive mass-spectrometer. Statistical analysis was performed by using R software with FactoMine R version 1.29 and ROCR version 1.0.5.

### EXAMPLE 2: Identification of Sex-Discriminant DNA Sequences in the External Layers of Chicken Eggs

Using PCR (Polymerase Chain Reaction) technique, we identified various DNA fragments differentially present between females and males in the external layers of the egg. The fragments identified and their specificity toward chicken gender, are listed in the Table 1. As an example CHD-W and CHD-Z fragments have been amplified and detected at different places in the external layer of the egg (Cuticle, Eggshell and External membrane) **(see** **Figure 2****).** Since there is an accumulation of total embryo-specific DNA in the eggshell with time, there is a very large panel of DNA that can be targeted to determine the sex of the embryo inside the egg **(****Figure 4****).**

**Liquid isothermal-PCR or regular PCR.** By using the natural porosity of the eggshell and notably the presence of pores, we envision to amplify sex-specific DNA at the surface of the egg without opening it. In the liquid reaction, all the reagents necessary for isothermal Polymerase Chain Reaction (PCR) or regular PCR are present. The direct contact between the liquid and the DNA targeted will allow its amplification. The amplification will be concomitant with the detection process. Different detection processes are envisioned: SybrGreen 1, Calcein or HNB Dye (Hydroxy Naphthol Blue) or beacon probes, direct DNA-binding and florescence. The Raman spectroscopy technology that has already been developed to sex chicken eggs (see Patent application WO 2016/005539, pages 9-16, paragraphs 6.1 and 6.2 and Figure 5) is also envisioned to detect specific biomarkers in the eggshell.

### EXAMPLE 3: Identification of Sex-Discriminant Peptides or Proteins, in the External Layers of Chicken Eggs

Using mass-spectrometry identification, we identified various peptides and proteins present in differential amounts between females and males in the external layers of the egg.

**Table 2: Summary of the peptides identified as differentially present between males and females chicken eggs at day 0 (D0).**

| SEQ ID Number | Peptide Sequence - Differential Females/Males at D0 | Corresponding protein (UniProt reference number) | Fold Change (F/M) | AUC |
|---|---|---|---|---|
| 19 | AspAspAspLysAsnLeuGlyAlaLeuCysGlyAr g | A0AID5PLK4 | 3.86 | 0.743 |
| 20 | CysAsnSerHisGlyTyrIleGluGluAlaCysIleIle ProCysProSerAspCysLys | F1NPY9 | 2.323 | 0.719 |
| 21 | ValTyrProGluThrGlnAlaGlnGluProGlyVal SerAlaSerLeuLys | A0A1D5P2R3 | 2.189 | 0.7 |
| 22 | ValProValThrAspHisSerLeuHisLeuSerGln AlaLys | F1NNS8 | 2.15 | 0.711 |
| 23 | SerTyrLysProProPheAspThrPheGluTyrPhe GlnAspLys | A0A1D5PBI7 | 2.143 | 0.71 |
| 24 | ThrLeuPheGlyGlnTyrGlyGlyGlySerPheSer PheSerArg | R4GF99 | 2.088 | 0.716 |
| 25 | ArgGlyGlyGlyProGlnAlaIleSerIleGlyLys | A0A1D5P755 | 0.496 | 0.701 |
| 26 | AspGlyAsnAlaSerGlyThrThrLeuLeuGlu AlaLeuAspCysIleLeuProProThrArgPro ThrAspLysProLeuArg | Q90835 | 0.451 | 0.719 |
| 27 | ThrGlnValGluThrLeuGluGluAspTyrThr PheMetAlaLeuSerLeuTrpLys | F1NFV7 | 0.375 | 0.705 |
| 28 | IleThrValValAlaAlaAspArg | F1NWW5 | 0.375 | 0.7 |
| 29 | LeuValGlnAlaPheGlnPheThrAspLys | P0CB50 | 0.328 | 0.767 |

**Table 3: Summary of the proteins identified as differentially present between males and females chicken eggs at day 0**

| Protein - Differential Females / Males at D0 (UniProt reference number). | Fold Change (F/M) | AUC |
|---|---|---|
| A0A1D5PHC7 | 1,162 | 0,702 |
| F1NNS8 | 1,131 | 0,702 |

**Table 4: Summary of the peptides identified as differentially present between males and females chicken eggs at day 9 post-incubation.**

| SEQ ID Number | Peptide Sequence Differential Females / Males at D9 (UniProt reference number). | Corresponding protein | Fold Change (F/M) | AUC |
|---|---|---|---|---|
| 30 | GluAspGluAsnIleLeuPheCysProLeuSerIleAla IleAlaMetGlyMetIleGluLeuGlyAlaHisGlyThr ThrLeuLys | Q90935 | 1.854 | 0.708 |
| 31 | CysProGlyGluSerSerGlnIleCysAspTrIleArg | F1NYK1 | 0.57 | 0.72 |
| 32 | GluLeuLysProThrValValAsnLeuAspPheGln Arg | R4GH41 | 0.528 | 0.72 |
| 33 | LeuHisLeuAspLeuAlaValAspValValAlaLys | F1NA58 | 0.51 | 0.707 |

**Table 5: Summary of the proteins identified as differentially present between males and females chicken eggs at day 9 post-incubation.**

| Protein - Differential Females / Males at D9 (UniProt reference number). | Fold Change (F/M) | AUC |
|---|---|---|
| A0A1L1RPA7 | 1.575 | 0.746 |
| A0A1D5P946 | 0.8 | 0.714 |
| F1NEZ4 | 0.782 | 0.743 |
| F1P0Z3 | 0.763 | 0.716 |
| Q90705 | 0.426 | 0.706 |

By combining different peptides or proteins we can predict the sex of the embryo present in the egg with satisfying specificity and sensitivity.

**Table 6: Summary of potential combination of peptidic biomarkers to predict egg sex at D0.**

| Peptides selected SEQ ID Number | AUC | Accuracy | Specificity | Sensibility |
|---|---|---|---|---|
| 27 and 22 | 0.827 | 77 | 68 | 86 |
| 23 and 20 | 0.821 | 77 | 86 | 68 |
| 17 and 26 | 0.825 | 75 | 64 | 86 |
| 17 and 25 | 0.806 | 82 | 82 | 82 |
| 18, 27 and 22 | 0.894 | 84 | 82 | 86 |
| 27, 23 and 20 | 0.878 | 86 | 91 | 82 |
| 27, 22 and 20 | 0.875 | 84 | 82 | 86 |
| 17, 26 and 20 | 0.874 | 82 | 86 | 77 |
| 27, 22 and 25 | 0.873 | 86 | 86 | 86 |

**Table 7: Summary of potential combination of peptidic biomarkers to predict egg sex at D9.**

| Peptide(s) selected SEQ ID Number | AUC | Accuracy | Specificity | Sensibility |
|---|---|---|---|---|
| 30 and 33 | 0.8 | 76.56 | 78.12 | 75 |
| 31 and 33 | 0.779 | 75 | 75 | 75 |
| 30 and 32 | 0.776 | 71.88 | 75 | 68.75 |
| 31 and 30 | 0.759 | 76.56 | 75 | 78.12 |
| 31 and 32 | 0.778 | 76.56 | 75 | 78.12 |
| 31, 30 and 33 | 0.831 | 84.38 | 90.62 | 78.12 |
| 30, 32 and 33 | 0.832 | 81.25 | 87.5 | 75 |
| 31, 30 and 32 | 0.811 | 79,69 | 81.25 | 78.12 |
| 31, 32 and 33 | 0.809 | 75 | 62.5 | 87.5 |
| 31, 30, 32 and 33 | 0.853 | 82.81 | 93.75 | 71.88 |

**Table 8: Summary of potential combination of proteic biomarkers to predict egg sex at D0.**

| Proteins selected (UniProt reference number). | AUC | Accuracy | Specificity | Sensibility |
|---|---|---|---|---|
| A0A1D5PHC7 | 0.702 | 66 | 59 | 73 |
| F1NNS8 | 0.702 | 68 | 64 | 73 |
| A0A1D5PHC7 + F1NNS8 | 0.778 | 77 | 82 | 73 |

**Table 9: Summary of potential combination of proteic biomarkers to predict egg sex at D9.**

| Markers (UniProt reference number). | AUC | Accuracy | Specificity | Sensibility |
|---|---|---|---|---|
| A0AILIRPA7 + F1NEZ4 | 0.807 | 78 | 78 | 78 |
| F1NEZ4 + F1P0Z3 | 0.82 | 78 | 75 | 81 |
| A0AILIRPA7 + F1P0Z3 | 0.797 | 77 | 84 | 69 |
| A0A1D5P946 + F1NEZ4 | 0.792 | 73 | 59 | 88 |
| A0A1D5P946 + A0AILIRPA7 | 0.787 | 75 | 75 | 75 |
| Q90705 + A0AILIRPA7 | 0.756 | 72 | 78 | 66 |
| Q90705 + F1P0Z3 | 0.767 | 72 | 69 | 75 |
| Q90705 + F1NEZ4 | 0.755 | 72 | 69 | 75 |
| A0A1D5P946 + Q90705 | 0.744 | 70 | 69 | 72 |
| A0A1D5P946 + F1P0Z3 | 0.731 | 72 | 56 | 88 |
| A0AILIRPA7 + F1NEZ4 + F1P0Z3 | 0.847 | 83 | 78 | 88 |
| A0A1D5P946 + A0A1L1RPA7 + FINEZ4 | 0.829 | 80 | 78 | 81 |
| Q90705 + F1NEZ4 + F1P0Z3 | 0.833 | 80 | 75 | 84 |
| Q90705 + A0A1L1RPA7 + F1P0Z3 | 0.818 | 78 | 75 | 81 |
| Q90705 + A0A1L1RPA7 + F1NEZ4 | 0.81 | 78 | 78 | 78 |
| A0A1D5P946 + F1NEZ4 + F1P0Z3 | 0.82 | 78 | 75 | 81 |
| A0A1D5P946 + Q90705 + F1NEZ4 | 0.802 | 75 | 59 | 91 |
| A0A1D5P946 + A0A1L1RPA7 + F1P0Z3 | 0.799 | 78 | 84 | 72 |
| A0A1D5P946 + Q90705 + A0AILIRPA7 | 0.804 | 75 | 75 | 75 |
| A0A1D5P946 + Q90705 + F1P0Z3 | 0.771 | 73 | 66 | 81 |
| Q90705 + A0AILIRPA7 + F1NEZ4 + F1P0Z3 | 0.856 | 84 | 78 | 91 |
| A0A1D5P946 + A0A1L1RPA7 + F1NEZ4 + F1P0Z3 | 0.847 | 83 | 84 | 81 |
| A0A1D5P946 + Q90705 + A0AILIRPA7 + F1NEZ4 | 0.835 | 81 | 75 | 88 |
| A0A1D5P946 + Q90705 + A0AILIRPA7 + F1P0Z3 | 0.82 | 78 | 69 | 88 |
| A0A1D5P946 + Q90705 + F1NEZ4 + F1P0Z3 | 0.833 | 80 | 75 | 84 |
| A0A1D5P946 + Q90705 + A0AILIRPA7 + F1NEZ4 + F1P0Z3 | 0.856 | 84 | 78 | 91 |

### Sex-Specific Peptide or Protein Identification

First, an acidic solution composed of HCl or Na-acetate is applied to the surface of the unhatched egg on the air chamber side to dissolve the calcium carbonate matrix. After dissolution of the eggshell parts between the cuticle and the mammillary cones (just before the external membrane), a buffered solution is applied to the surface to bring the surface pH at 7. Next, a solution comprising all the components necessary for the extraction of protein or peptide is applied to the surface. After an incubation time that can vary from 1 minute to 1 hour, the solution is aspirated and analysed to detect the biomarker of interest. For protein or peptide detection, two techniques are envisioned: liquid chromatography coupled with mass-spectrometry analyses or specific antibody detection by ELISA. Many commercially-available antibodies can be used: anti-AGRN monoclonal antibody, clone 6D2 (Company Creative Diagnostics) for A0A1D5PHC7; PRDX4 / Peroxiredoxin 4 antibody (aa201-25 LS-BIO) for F1NNS8; Chicken type I collagen antibody (Novotec) for A0A1D5PLK4; anti-SFRP1 antibody (antibodies-online) for A0A1D5P2R3; Neuroserpin antibody (Biorbyt) for Q90935; SERPINH1 antibody (antibodies-online) for F1NA58; EEF2 / Elongation Factor 2 antibody (LS Bio) for Q90705. The Raman spectroscopy technology that has already been developed to sex chicken eggs (Patent WO 2016/005539) is also envisioned to detect specific biomarkers in the eggshell.

### EXAMPLE 4: Identification of Peptides or Proteins in the External Layers of Chicken Eggs, indicating the level of maturity of the embryo.

Using mass-spectrometry identification, we also identified various peptides and proteins present in differential amounts between eggs at days 0 and days 9 of the embryonic development in the external layers of the egg.

**Table 10: Summary of the peptides identified as differentially present between eggs at days 0 and 9 of their embryonic development.**

| SEQ ID Number | Peptide Sequence - Differential D9/D0 | Corresponding protein | Fold Change (D9/D0) | AUC |
|---|---|---|---|---|
| 34 | IleAlaGlyHisAlaGluGluTyrGlyAlaGluThrLeu GluArg | P01994 | 817 | 0.96 |
| 35 | GlyAlaThrTyrAsnIleIleValGluAlaLeuLysAsp HisArgArg | F1NJT3 | 669 | 0.94 |
| 36 | ValLeuThrSerPheGlyAspAlaValLys | P02112 | 594 | 0.94 |
| 37 | LeuSerProSerPheGlnGlyHisValCysGlyLeu CysGlyAsnTyrAspGlyAsnGlyAsnAsnAspPhe | A0A1D5NT78 | 0.5 | 0.93 |
| | ThrThrArg | | | |
| 38 | SerArgProIleLeuProIleTyrLeuLys | A0AID5PI58 | 0.18 | 0.93 |
| 39 | AlaLeuHisPheAspSerIleAlaGlyLeuGlyGly SerThrGlnThrLys | A0AID5PI58 | 0.15 | 0.95 |
| 40 | GluLeuLeuSerAspIleThrAlaSerLys | A0AID5PI58 | 0.13 | 0.92 |
| 41 | His AsnProThr AsnThrlleV alT yrPheGly Arg | P01013 | 0.13 | 0.92 |
| 42 | PheGlyValAsnGlySerGluLys | A0A1D5P4L7 | 0.0002 | 0.98 |
| 43 | ProGlnAlaProIleThrGlyTyrArg | F1NJT3 | Not present at day9 | 0.93 |

**Table 11: Summary of the proteins identified as differentially present between eggs at days 0 and 9 of their embryonic development.**

| Protein - Differential D9 / D0 (UniProt reference number). | Fold Change (D9 / D0) | AUC |
|---|---|---|
| P02112 | 549.476 | 0.95 |
| P01994 | 262.55 | 0.89 |
| P02701 | 0.414 | 0.88 |
| A0A1D5PI58 | 0.236 | 0.95 |
| P41366 | 0.002 | 0.92 |

**Table 12: Summary of potential combination of peptidic biomarkers to predict egg embryo maturity.**

| Peptidic Marker (SEQ ID Number) | AUC | Accuracy | Specificity | Sensibility |
|---|---|---|---|---|
| 43 and 39 | 0.998 | 98.15 | 100 | 96.88 |
| 42 and 43 | 0.998 | 97.22 | 100 | 95.31 |
| 42 and 35 | 0.997 | 99.07 | 100 | 98.44 |
| 43 and 40 | 0.997 | 97.22 | 100 | 95.31 |
| 42, 34 and 43 | 1 | 99 | 100 | 98 |
| 34,43 and 39 | 1 | 99 | 100 | 98 |
| 3, 43 and 37 | 1 | 98 | 100 | 97 |
| 43, 39 and 35 | 1 | 99 | 98 | 100 |
| 43, 35 and 40 | 1 | 99 | 98 | 100 |

**Table 13: Summary of potential combination of proteic biomarkers to predict egg embryo maturity.**

| Markers (UniProt reference number). | AUC | Accuracy | Specificity | Sensibility |
|---|---|---|---|---|
| P02112 + A0A1L1RJ91 | 0.99 | 95 | 98 | 94 |
| P01994 + AOA1L1RJ91 | 0.98 | 92 | 100 | 86 |
| A0A1D5PI58 + P02112 | 0.96 | 93 | 98 | 89 |
| A0A1D5PI58 + P01994 | 0.96 | 93 | 98 | 89 |
| A0A1D5PI58 + P02127 | 0.96 | 92 | 98 | 88 |
| P01994 + AOA1L1RJ91 + P02127 | 0.99 | 95 | 100 | 92 |
| P02112 + AOA1L1RJ91 + P02127 | 0.98 | 94 | 95 | 94 |
| A0A1D5PI58 + P01994 + P01012 | 0.98 | 94 | 98 | 91 |
| A0A1D5PI58 + P02112 + P01012 | 0.98 | 94 | 95 | 92 |
| A0A1D5PI58 + P02127 + P01012 | 0.98 | 94 | 91 | 97 |

Proteins or peptides of interest have been selected by their fold change ratio between males and females at days 0 or 9 after laying day or by their fold change ratio between days 0 or 9 after laying day. To be selected by our testes, a biomarker must have a fold change ratio superior to 2 or inferior to 0.5. Moreover we selected the ones that have an AUC of at least 0.7 with a preference for the ones having an AUC superior to 0.75. Once we combine 2 or more biomarker to determine a characteristic of the embryo inside the egg, our testes are more performants. Some combinations give AUCs superior to 0.8 and accuracy, specificity and sensibility better than 80%. They are the ones we will use to determine the gender or the stage of development of the unhatched embryos.

### EXAMPLE 5: Identification of Sex-Discriminant Metabolites in the External Layers of Chicken Eggs.

Using mass-spectrometry identification, we identified the glucose that is present at differential amounts between females and males in the external layers of the egg. **Table 14** Summary of potential metabolic biomarker to predict egg sex.

The glucose is present at differential amounts between females and males in the external layers of the egg. (see Table 14)

**Table 14**

| Metabolic Biomarker | Test at D0 | |
|---|---|---|
| Glucose | Fold Change (F/M) | p-value |
| | 2.680 | 0.058 |

### C) Sex-Specific "in External Layers" Metabolite Identification

First, an acidic solution composed of HCl or Na-acetate is applied to the surface of the unhatched egg on the air chamber side to dissolve the calcium carbonate matrix. After dissolution of the eggshell parts between the cuticle and the mammillary cones (just before the external membrane), a buffered solution is applied to the surface to bring the surface pH at 7. Next, a solution comprising all the components necessary for the extraction of metabolites is applied to the surface. After an incubation time that can vary from 1 minute to 1 hour, the solution is aspirated and analysed to detect the biomarker of interest. For metabolite detection, specific biochemical reactions that process the metabolite of interest coupled with detection methods will be used. The Raman spectroscopy technology that has already been developed to sex chicken eggs (Patent application published under the number WO 2016/005539) can be also envisioned to detect specific biomarkers in the eggshell.

## Claims

1. A method for determining whether a compound present at the surface of, or in the eggshell is a biomarker (or a biomarker combination) specific of a characteristic of an embryo in an unhatched egg, said method comprising the steps of:
a) determining the presence of at least said compound in or on the eggshell, said measure being carried out at different time periods from the incubation until to the hatching of the egg;
b) optionally measuring the amount of said compound in or on the eggshell, said measure being carried out at different time periods of from the incubation until to the hatching of the egg;
c) comparing the results obtained in step a) and, optionally in step b), particularly the decrease or the increase of said compound amount obtained in step b) function of the location of the analysed egg compound, to a base line established for said compound for positive and negative controlled embryos for said characteristic;
d) determining whether said compound is a biomarker specific of a characteristic of the embryo,

2. A method for the determination of a characteristic of an embryo in an unhatched egg, said method comprising the steps of:
a) detecting and/or measuring the amount of at least a known biomarker specific of said characteristic at a time period of from the incubation from the egg laying until to the hatching of the egg;
b) comparing the amount obtained in step a) to a base line established for said biomarker at the same time incubation period for positive and negative controlled embryos for said characteristic;
c) determining whether the embryo possesses said characteristic by analysing the results obtained in step b),
and wherein the amount of said biomarker is detected and/or measured at the surface or in the eggshell of the egg.

3. The method according to claim 2, wherein said compound which is detected and/or measured is selected by the method of claim 1.

4. The method according to anyone of claims 1 to 3, wherein the method is a non-destructive method.

5. The method according to anyone of claims 1 to 4, wherein in step a), the eggshell inner membrane is not penetrated when the tested compound is detected and/or measured.

6. The method according to anyone of claims 1 to 5, wherein said compound which is measured is selected from the group consisting of nucleic acids, proteins, peptides and metabolites.

7. The method according to anyone of claims 1 to 6, wherein said egg is selected from the group of oviparous animals, including birds.

8. The method of anyone of claim 1 to 7, wherein said embryo characteristic which is desired to determine is selected from the group consisting of gender, fertility, stages of development, viability or any genetic or metabolic characteristics of interest.

9. The method of claim 8, wherein said embryo characteristic which is desired to determine is the gender of the embryo.

10. The method of one of claims 1 to 9, wherein said compound is a nucleic acid biomarker compound, preferably DNA fragment, whose presence or concentration is specific of the embryo and selected from the group consisting of HINTW, CHD1-W, CHD1-Z, AC239603.4, AC182258.2, AF461428.1, SWIM, ATP5A1W, Spin-W, ASW, Sox9, CYP19A1, AMH (Anti-Mullerian hormone), FET1(female expressed transcript; trans-membrane domain), SF1, Dax, 12S, 18S, preferably nucleic acid biomarker whose presence or concentration is specific of the embryo gender and selected from the group consisting of CHD1-W, AC239603.4, AC182258.2, AF461428.1, SWIM, HINTW, ATP5A1W, Spin-W, ASW, Sox9, CYP19A1, AMH (Anti-Mullerian hormone), FET1 (female expressed transcript; trans-membrane domain), SF1 and Dax .

11. The method of one of claims 1 to 9, wherein said compound is a protein or peptidic biomarker compound selected from the group consisting of the proteins A0A1D5PHC7, F1NNS8, A0A1L1RPA7, A0A1D5P946, F1NEZ4, F1P0Z3, Q90705, P02112, P01994, P02701, A0A1D5PI58, P41366 or specific fragments thereof having at least 12 amino acids and the peptides having or comprising the sequences SEQ ID N0.: 19 to 43, preferably protein, or specific fragments thereof, whose presence or concentration is specific of the embryo gender and selected from the group consisting of the proteins A0A1D5PHC7, F1NNS8, A0A1L1RPA7, A0A1D5P946, F1NEZ4, F1P0Z3, Q90705 and the peptides SEQ ID N0.:19 to 43, or a combination of at least 2, preferably 3 or 4 of these proteins or peptides, or fragments thereof.

12. The method of one of claims 1 to 9, wherein said compound is a metabolite biomarker preferably metabolite, whose presence or concentration is specific of the embryo gender and selected from the group consisting of glucose.

13. The method of claim 10, wherein said specific DNA fragment allowing the gender determination of the egg is selected from the group of W-linked genic or intergenic regions CHD1-W, AC239603.4, AC182258.2, AF461428.1, SWIM, HINTW, ATP5A1W, Spin-W, ASW, Sox9, CYP19A1, AMH (Anti-Mullerian hormone), FET1 (female expressed transcript; trans-membrane domain), SF1, Dax.

14. The method of claim 13, wherein said specific DNA fragment allowing the gender determination of the egg is a DNA fragment having or comprising a sequence selected from the group of the sequences SEQ ID NO.: 13 to 18 or sequences of specific fragments thereof having at least 12, preferably 15 nucleotides.

15. The method of claim 14, wherein said DNA fragment specific of said chromosome is a DNA fragment comprising at least the sequence SEQ ID No.13 or 14 or 15 or 16 preferably the sequence SEQ ID No.13 or 15.
